# EUROPÄISCHE PATENTSCHRIFT

(11) **EP 1 761 510 B1**
(45) Veröffentlichungstag und Bekanntmachung des Hinweises auf die Patenterteilung: **13.05.2015**
(21) Anmeldenummer: 05769631.2
(22) Anmeldetag: 18.06.2005
(51) Int. Cl.: C07D 413/14, C07D 271/07, C07D 401/12, A61K 31/4184, A61P 7/02

(54) **VERFAHREN ZUR HERSTELLUNG VON 4-(BENZIMIDAZOLYLMETHYLAMINO)- BENZAMIDINEN**
METHOD FOR PRODUCING 4-(BENZIMIDAZOLYLMETHYLAMINO)-BENZAMIDINES
PROCÉDÉ POUR PRODUIRE DES 4-(BENZIMIDAZOLYLMETHYLAMINO)-BENZAMIDINES

(30) Priorität: 25.06.2004 EP 04014917
(43) Veröffentlichungstag der Anmeldung: 14.03.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: ZERBAN, Georg, 55218 Ingelheim (DE); HAUSHERR, Arndt, 55122 Mainz (DE); SCHLARB, Kerstin, 55606 Baerweiler (DE); SCHMITT, Heinz-Peter, 55218 Ingelheim (DE); WEYELL, Björn, 55459 Aspisheim (DE); KOCH, Gunter, 5270 Schwabenheim (DE); HAMM, Rainer, 55218 Ingelheim (DE)
(74) Vertreter: Simon, Elke Anna Maria
(86) Internationale Anmeldenummer: PCT/EP2005/006586
(87) Internationale Veröffentlichungsnummer: WO 2006/000353

(56) Entgegenhaltungen:
- WO-A-00/01704
- WO-A1-98/37075
- WO-A1-2005/090382
- DE-A- 19 962 329

## Beschreibung

### HINTERGRUND DER ERFINDUNG

### 1. TECHNISCHES GEBIET

Die Erfindung betrifft ein Verfahren zur Herstellung eines gegebenenfalls substituierten 4-(Benzimidazol-2-ylmethylamino)-benzamidins, wobei man
(a) ein gegebenenfalls entsprechend substituiertes Diaminobenzol mit 2-[4-(1,2,4-Oxadiazol-5-on-3-yl)-phenylamino]-essigsäure kondensiert,
(b) das so erhaltene Produkt hydriert, und
(c) gegebenenfalls die Amidinogruppe carbonyliert.

### 2. STAND DER TECHNIK

Substituierte (4-Benzimidazol-2-ylmethylamino)-benzamidine, insbesondere 1-Methyl-2-[*N*-[4-(*N*-n-hexyloxycarbonylamidino)phenyl]-amino-methyl]-benzimidazol-5-yl-carbonsäure-*N*-(2-pyridyl)-*N*-(2-ethoxycarbonylethyl)-amid sind bereits aus der Internationalen Patentanmeldung WO 98/37075 als Wirkstoffe mit einer Thrombin-hemmenden und die Thrombinzeit verlängernden Wirkung offenbart werden, bekannt.

Hauptindikationsgebiet der Verbindung der chemischen Formel I ist die postoperative Prophylaxe von tiefen Venenthrombosen.

In der WO 98/37075 wird vorgeschlagen die substituierten (4-Benzimidazol-2-ylmethylamino)-benzamidine durch Umsetzung der entsprechenden, substituierten (4-Benzimidazol-2-ylmethylamino)-benzonitrile mit Ammoniak herzustellen. Dieses Verfahren ist produktionstechnisch sehr aufwändig und führt zu einer hohen Belastung an zu entsorgenden Säuren.

Der vorliegenden Erfindung lag die Aufgabe zu Grunde eine Verfahrensvariante zur Herstellung der substituierten (4-Benzimidazol-2-ylmethylamino)-benzamidine aufzuzeigen, bei der diese produktionstechnisch aufwändige Verfahrensstufe vermieden werden kann.

### KURZE ZUSAMMENFASSUNG DER ERFINDUNG

Überraschenderweise wurde gefunden, dass substituierte 4-(Benzimidazol-2-ylmethylamino)-benzamidine in hohen Ausbeuten und unter Einsatz kostengünstiger Hilfsstoffe hergestellt werden können, wenn man
(a) ein gegebenenfalls entsprechend substituiertes Diaminobenzol mit 2-[4-(1,2,4-Oxadiazol-5-on-3-yl)-phenylamino]-essigsäure kondensiert,
(b) das so erhaltene Produkt hydriert, und
(c) gegebenenfalls die Amidinogruppe, vorzugsweise mit einem Alkylhalogenformiat in Gegenwart einer Base, insbesondere mit Hexylchloroformiat carbonyliert.

Gegenstand der Erfindung ist deshalb ein Verfahren zur Herstellung eines gegebenenfalls substituierten 4-(Benzimidazol-2-ylmethylamino)-benzamidins der Formel (I) 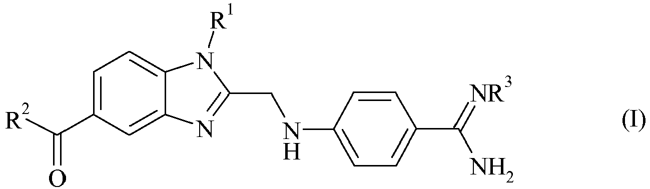 worin
R¹ für eine Methylgruppe steht,
R² für eine R²¹NR²²-Gruppe steht, in der
   R²¹ eine Ethylgruppe, die durch eine Ethoxycarbonylgruppe substituiert ist, bedeutet, und
   R²² Pyridin-2-ylgruppe bedeutet,
R³ für eine Hexyloxycarbonylgruppe steht; 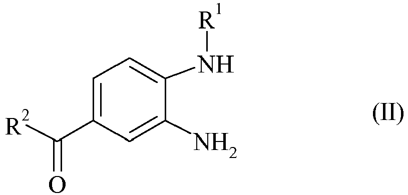
wobei man im Schritt (a) ein Phenyldiamin der Formel (II)
worin R¹ und R² die für Formel (I) angegebene Bedeutung aufweisen; mit 2-[4-(1,2,4-Oxadiazol-5-on-3-yl)-phenylamino]-essigsäure umsetzt;
das erhaltene Produkt der Formel (III) 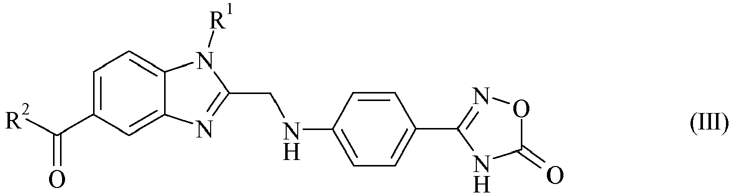 worin R¹ und R² die für Formel (I) angegebene Bedeutung aufweisen,
in Schritt (b) hydriert, und
(c) gegebenenfalls die so erhaltene Verbindung der Formel (I), worin R³ Wasserstoff bedeutet, mit einer Verbindung der Formel (IV)

   R³-X (IV)
worin R³ die für Formel (I) angegebene Bedeutung aufweist, und
X für eine geeignete Abgangsgruppe steht,
umsetzt.

Ein weiterer Gegenstand der Erfindung sind die bei dem erfindungsgemäßen Verfahren durchlaufenen, neuen Zwischenprodukte der Formel III: 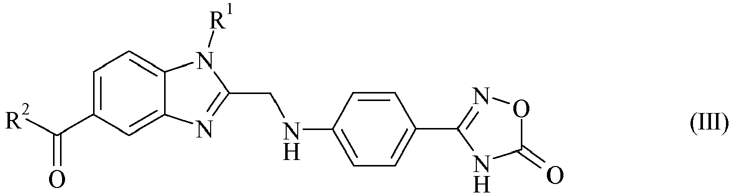 worin R¹ und R² für die nachfolgende Verbindungen der Formel (I) angegebene Bedeutung aufweisen, sowie 2-[4-(1,2,4-Oxadiazol-5-on-3-yl)-phenylamino]-essigsäure.

### DETAILLIERTE BESCHREIBUNG DER ERFINDUNG

Die Ausführungsformen (A) bis (D) des erfindungsgemäßen Verfahrens sind bevorzugt:
(A) Die Kondensation des Schrittes (a) wird in Gegenwart eines inerten Verdünnungsmittels und eines wasserbindenden Mittels durchgeführt.

Die entsprechend substituierten Diaminobenzole der Formel (II) sind z.B. aus der Internationalen Patentanmeldung WO 98/37075 bekannt oder können in Analogie zu den dort beschriebenen hergestellt werden. Besonders bevorzugt wird 3-Amino-4-methylaminobenzoesäure-*N*-(2-pyridyl)-*N*-(2-ethoxycarbonylethyl)-amid eingesetzt.

Als inerte Verdünnungsmittel können sowohl aprotische apolare Lösungsmittel - wie z.B. aliphatische oder aromatische, gegebenenfalls halogenierte Kohlenwasserstoffe - oder aprotische polare Lösungsmittel wie z.B. Ether und/oder Amide bzw. Lactame und/oder Gemische davon eingesetzt werden. Als aprotische apolare Lösungsmittel werden bevorzugt verzweigte oder unverzweigte C₅ - C₈ aliphatische Alkane, C₄ - C₁₀ Cycloalkane, C₁ - C₆ aliphatische Halogenalkane, C₆ - C₁₀ aromatische Alkane oder Gemische davon eingesetzt. Besonders bevorzugt werden Alkane wie Pentan, Hexan oder Heptan, Cycloalkane wie Cyclohexan oder Methylcyclohexan, Halogenalkane wie Dichlormethan, aromatische Alkane wie Benzol, Toluol oder Xylol oder deren Mischungen eingesetzt. Als aprotische Lösungsmittel eignen sich polare Ether wie beispielsweise Tetrahydrofuran (THF), *tert*-Butyl-Methylether oder Dimethoxyethylether oder Amide wie beispielsweise Dimethylformamid, oder Lactame wie beispielsweise N-Methylpyrro lidon.

Als wasserbindende Mittel können hygroskopische Salze, anorganische oder organische Säuren, Anhydride von anorganischen oder organischen Säuren, Molsiebe oder Harnstoffderivate eingesetzt werden. Besonders bevorzugt ist 1,1'-Carbonyldiimidazol.

In einer besonders bevorzugten Ausführungsform wird 1,1'-Carbonyldiimidazol in THF suspendiert und erwärmt. 2-[4-(1,2,4-Oxadiazol-5-on-3-yl)-phenylamino]-essigsäure wird zugegeben. Das entsprechend substituierte Diaminobenzol wird in THF hinzugefügt. Die Reaktionsmischung wird bei etwa 50°C gerührt und anschließend nach Zugabe von Essigester eingeengt.
(B) Die Hydrierung des Schrittes (b) wird in Gegenwart eines inerten Verdünnungsmittels und eines Hydrierkatalysators durchgeführt.

Besonders bevorzugt ist ein Verfahren, wobei die Hydrierung in einem Temperaturbereich von 0°C bis 100°C, vorzugsweise von 0°C bis 50°C, insbesondere von 10°C bis 30°C durchgeführt wird.

Weiterhin bevorzugt ist ein Verfahren, wobei die Hydrierung unter einem Druck von mehr als 0,5 bar bis 100 bar, vorzugsweise unter einem Druck von 1 bar bis 10 bar, insbesondere bei etwa 1 - 2 bar durchgeführt wird.

Als inerte Verdünnungsmittel können sowohl protische Lösungsmittel - wie z.B. Alkohole, Carbonsäuren und/oder Wasser - oder aprotische polare Lösungsmittel wie z.B. Ether und/oder Amide bzw. Lactame und/oder Gemische davon eingesetzt werden. Allen Lösungsmitteln kann gegebenenfalls Wasser zugesetzt sein. Als protische Lösungsmittel werden bevorzugt verzweigte oder unverzweigte C₁ - C₈ Alkanole, C₁ - C₃ Carbonsäuren oder Gemische davon eingesetzt. Besonders bevorzugt werden niedere Alkohole wie Methanol, Ethanol, n-Propanol und Isopropanol, Carbonsäuren wie Ameisensäure, Essigsäure und Propionsäure oder deren Mischungen eingesetzt. Besonders bevorzugt wird als Reaktionsmedium Ethanol und/oder Essigsäure verwendet, wobei diese gegebenenfalls Wasser enthalten können. Als aprotische Lösungsmittel eignen sich polare Ether wie beispielsweise Tetrahydrofuran oder Dimethoxyethylether oder Amide wie beispielsweise Dimethylformamid, oder Lactame wie beispielsweise N-Methylpyrrolidon. Bevorzugt werden Lösungsmittel eingesetzt, die wenig zur Brennbarkeit neigen.

Als Hydrierkatalysatoren sind in der Regel Übergangsmetalle wie zum Beispiel Nickel, Platin oder Palladium oder deren Salze oder Oxide geeignet. Bevorzugt sind Raney Nickel, Platinoxid und Palladium auf einem inerten Trägermaterial, insbesondere Palladium auf Aktivkohle (Pd/C).

Bevorzugt sind solche Verfahren, wobei man das Produkt des Schrittes (a) zum Hydrierkatalysator bei der Hydrierung in einem Gew.-Verhältnis von 1:1 bis 1000:1, vorzugsweise von 5:1 bis 100:1 einsetzt.

In einer besonders bevorzugten Ausführungsform wird das Produkt des Schritts (a) in Ethanol aufgenommen und nach Zugabe von Essigsäure und bei 2 bar Wasserstoff mit wasserfeuchtem 10%-igem Pd/C bei Raumtemperatur hydriert. Man filtriert vom Katalysator und gibt p-Toluolsulfonsäure, gelöst in 90 ml Ethanol zu. Das Tosylat des erhaltenen 4-(Benzimidazol-2-ylmethylamino)-benzamidins fällt aus, wird abfiltriert und in mehreren Portionen mit Ethanol nachgewaschen.
(C) Zur Herstellung von 2-[4-(1,2,4-Oxadiazol-5-on-3-yl)-phenylamino]-essigsäure wird 2-[4-(1,2,4-Oxadiazol-5-on-3-yl)-anilin mit einem 2-Halogenessigsäureester, vorzugsweise Bromessigsäureethylester, in Gegenwart einer schwachen Base, vorzugsweise ein tertiäres Amin, wie zum Beispiel Triethylamin oder ein Alkalimetallcarbonat, wie zum Beispiel Natriumcarbonat in einem inerten Lösungsmittel umgesetzt, und der erhaltene 2-[4-(1,2,4-Oxadiazol-5-on-3-yl)-phenylamino]-essigsäureester verseift.

Als inerte Verdünnungsmittel können sowohl protische Lösungsmittel - wie z.B. Alkohole, und/oder Wasser - oder aprotische polare Lösungsmittel wie z.B. Ether und/oder Amide bzw. Lactame und/oder Gemische davon eingesetzt werden. Allen Lösungsmitteln kann gegebenenfalls Wasser zugesetzt sein. Als protische Lösungsmittel werden bevorzugt verzweigte oder unverzweigte C₁ - C₈ Alkanole oder Gemische davon eingesetzt. Besonders bevorzugt werden niedere Alkohole wie Methanol, Ethanol, n-Propanol und Isopropanol oder deren Mischungen eingesetzt. Besonders bevorzugt wird als Reaktionsmedium Isopropanol verwendet, wobei dieses gegebenenfalls Wasser enthalten kann. Als aprotische Lösungsmittel eignen sich polare Ether wie beispielsweise Tetrahydrofuran oder Dimethoxyethylether oder Amide wie beispielsweise Dimethylformamid, oder Lactame wie beispielsweise N-Methylpyrrolidon.

In einer besonders bevorzugten Ausführungsform wird Bromessigsäureethylester zu einer Suspension von 2-[4-(1,2,4-Oxadiazol-5-on-3-yl)-anilin und Natriumcarbonat in Wasser/Isopropanol zudosiert und gerührt. Die gekühlte Suspension wird abgesaugt, in mehreren Portionen mit Wasser und Ethanol nach gewaschen und getrocknet.

Die Verseifung erfolgt vorzugsweise in einem protischen Lösungsmittel mit einem Alkali oder Erdalkalihydroxid, insbesondere mit Lithium-, Natrium oder Kaliumhydroxid.

In einer besonders bevorzugten Ausführungsform wird 2-[4-(1,2,4-Oxadiazol-5-on-3-yl)-phenylamino]-essigsäureester in Wasser suspendiert und bei Raumtemperatur langsam mit einer wässrigen Lösung von NaOH versetzt. Die Suspension geht in eine Lösung über und wird auf 45 bis 75°C erwärmt. Die so erhaltene Lösung wird mit HCl versetzt, bis etwa pH 5 erreicht ist. Der Feststoff wird isoliert und mit kaltem Wasser sowie kaltem Ethanol und MtBE gewaschen.
(D) Zur Herstellung von 2-[4-(1,2,4-Oxadiazol-5-on-3-yl)-anilin wird 4-Aminophenyl-amidoxim mit einem Dialkylcarbonat, vorzugsweise Dimethylcarbonat oder Diethylcarbonat in Gegenwart einer Base, vorzugsweise eines Alkalimetallalkoholats, insbesondere Natriummethylat, Natriumethylat oder Kalium *tert*-Butanolat umgesetzt.

4-Aminophenyl-amidoxim kann z.B. durch Umsetzung von 4-Aminobenzonitril mit Hydroxylamin Hydrochlorid hergestellt werden.

In einer besonders bevorzugten Ausführungsform wird Natriummethylat bei 70-75°C zu einer Suspension von 4-Aminophenyl-amidoxime in Ethanol gegeben und mit Ethanol nachgespült. Nach 15 min Rühren wird Diethylcarbonat zugetropft. Nach 2-4 Stunden Reaktionszeit wird abgekühlt und Ethanol bei 120 mbar und 40°C abdestilliert. Der Rückstand wird in Wasser aufgenommen nach Erwärmen durch halb konz. Natronlauge auf pH 10-12, dann durch Ansäuern mit konz. Salzsäure auf pH 5-6 eingestellt und langsam abgekühlt. Die Lösung geht in eine Suspension über, die filtriert und mehrmals mit kaltem Wasser und Ethanol nach gewaschen wird.

Die Herstellung der als Zwischenprodukt benötigten 2-[4-(1,2,4-Oxadiazol-5-on-3-yl)-phenylamino]-essigsäure aus 4-Aminobenzonitril wird im nachfolgenden Reaktionsschema dargestellt:

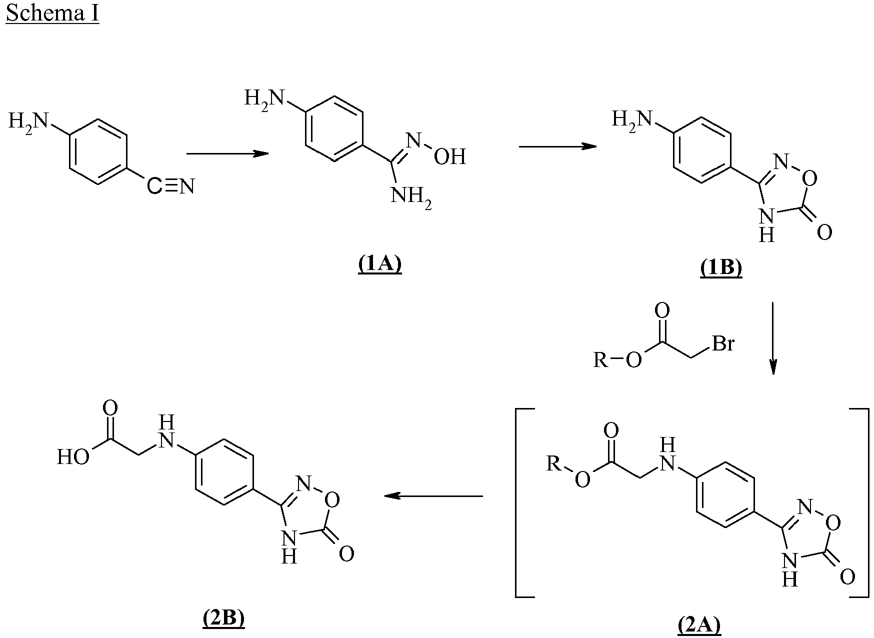

Die Herstellung eines 4-(Benzimidazol-2-ylmethylamino)-benzamidins wird beispielhaft im folgenden Reaktionsschema dargestellt:

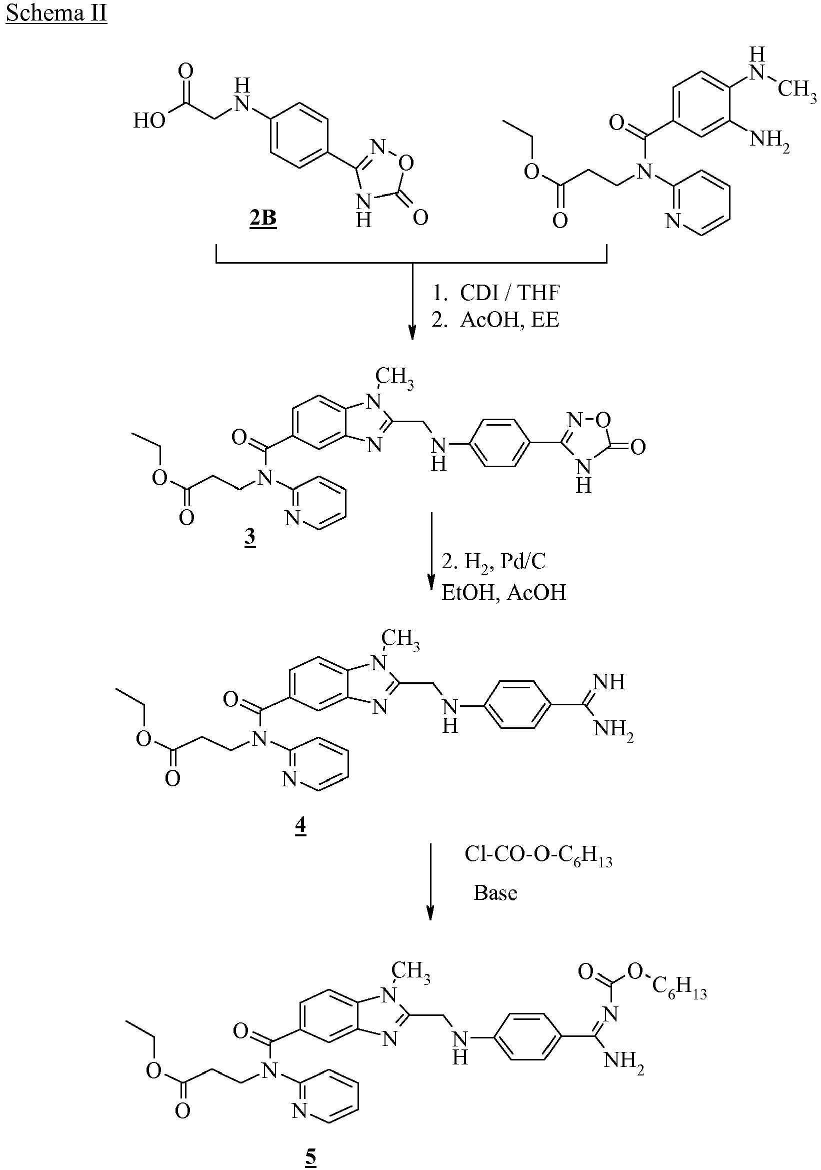

Die Aufarbeitung der einzelnen Reaktionen kann in üblicher Weise erfolgen, zum Beispiel, indem man die Reaktionshilfsmittel abtrennt, das Lösungsmittel entfernt und aus dem Rückstand reines Endprodukt durch Kristallisation, Destillation, Extraktion oder Chromatographie isoliert.

Das erfindungsgemäße Verfahren soll nun durch die nachfolgenden Beispiele erläutert werden. Dem Fachmann ist bewusst, dass die Beispiele nur zur Veranschaulichung dienen und als nicht limitierend anzusehen sind.

### BEISPIELE

Vor- und nachstehend werden folgende Abkürzungen verwendet:
- AcOH: Essigsäure
- AMBPA: 3-Amino-4-methylaminobenzoesäure-*N*-(2-pyridyl)-*N*-(2-ethoxycarbonylethyl)-amid
- CDI: 1,1'-Carbonyldiimidazol
- EE: Essigsäureethylester
- EtOH: Ethanol
- HCl: Salzsäure
- MtBE: Methyl-*tert*-Butylether
- NaOH: Natriumhydroxid
- PTSA: p-Toluolsulfonsäure
- RT: Raumtemperatur
- THF: Tetrahydrofuran

### Beispiel 1 - Herstellung von 2-[4-(1,2,4-Oxadiazol-5-on-3-yl)-anilin (1):

(1A) - Im Reaktionsgefäß werden 118,6 g (1 mol) 4-Aminobenzonitril und 68,9 g (0,65 mol) Natriumcarbonat in 500 ml Ethanol und 100 ml Wasser vorgelegt und auf 60°C erwärmt. 76,4 g (1,1 mol) Hydroxylamin Hydrochlorid, gelöst in 100 ml Wasser, werden langsam zu dieser Suspension zugetropft.

Die Mischung wird anschließend über Nacht bei 60°C gerührt. Beim Abkühlen auf 0-5°C fällt die Substanz aus, wird ab filtriert und mehrmals mit insgesamt 150 ml kaltem Wasser und 100 ml kaltem Ethanol nach gewaschen. Abschließend wird mit 50 ml MtBE gewaschen und man erhält 178,4 g feuchtes Produkt. Dieses wird bei 35°C im Vakuum getrocknet.
Ausbeute: 135,4 g hellbeige Substanz (89,5%d.Th), Schmelzpunkt: ab 169,5°C (Zers.); Reinheit: > 98% HPLC-Peakfläche

**(1B) -** Zu einer Suspension von 60,5 g **(1A)** (0,4 mol) in 400 ml Ethanol werden portionsweise bei 70-75°C 25,02 g (0,46 mol) Natriummethylat zugegeben und mit 20 ml Ethanol nachgespült.

Nach 15 min Rühren werden 47,25 g (0,4 mol) Diethylcarbonat zugetropft. Nach 3 Stunden Reaktionszeit wird auf 40°C abgekühlt und der Ethanol bei 120 mbar und 40°C abdestilliert. Man erhält einen dunklen Rückstand. Dieser wird bei 40-45°C in 350 ml Wasser gelöst und nach Aufheizen auf 70°C zuerst durch langsame Zugabe von halb konz. Natronlauge auf pH 11; dann durch Ansäuern mit konz. Salzsäure auf pH 5,5 eingestellt und langsam abgekühlt. Die Lösung geht in eine Suspension über, die filtriert und mehrmals mit insgesamt 150 ml kaltem Wasser und 50 ml Ethanol nach gewaschen wird.

Man erhält 88,7 g feuchte Substanz, die bei 35°C im Vakuum getrocknet wird.
Ausbeute: 62 g dunkle Substanz (87,5% d.Th.); Schmelzpunkt: ab 178°C (Zers.);
Reinheit: > 98% HPLC-Peakfläche

### Beispiel 2 - Herstellung von 2-[4-(1,2,4-Oxadiazol-5-on-3-yl)-phenylamino]-essigsäure (2):

**2(A)** - Bei Raumtemperatur werden 83,5 g (0,5 mol) Bromessigsäureethylester zu einer Suspension von 70,86 g (0,4 mol) **(1B)** und 26,5 g (0,25 mol) Natriumcarbonat in 600 ml Wasser/Isopropanol zudosiert und über Nacht gerührt. Das Reaktionsgemisch wird rötlichbraun bis orange.

Die auf 0°C abgekühlte Suspension wird abgesaugt, in mehreren Portionen mit 300 ml Wasser und 150 ml Ethanol nach gewaschen (106 g feuchte hellbraune Substanz) und bei 35°C im Vakuum getrocknet.

Ausbeute: 92,44 g bräunliche Substanz (87,7% d.Th.) Schmelzpunkt: ab 186,1°C (Zers.) Reinheit: > 98% HPLC-Peakfläche

**2(B)** - Der so erhaltene Ester **(2A)** (86,9 g; 0,33 mol) wird in 400 ml Wasser suspendiert und bei RT werden langsam 120 g 45%ige NaOH zugetropft. Die Suspension geht in Lösung und wird rötlich (pH 12,5). Es wird auf ∼60°C erwärmt und 1 h verseift. Die erhaltene Lösung wird portionsweise mit 37%iger HCl versetzt, bis pH 5 erreicht ist. Es wird auf 0°C abgekühlt. Der Feststoff wird abgesaugt und in mehreren Portionen mit insgesamt 400 ml kaltem Wasser sowie je 40 ml kaltem Ethanol und MtBE gewaschen. Man erhält 81,4 g feuchte dunkle Substanz. Es wird bei 35°C im Vakuum getrocknet.

Ausbeute: 76,7 g Substanz (98% d.Th.) Schmelzpunkt: ab 193°C (Zers.) Reinheit: > 99% HPLC-Peakfläche

### Beispiel 3 - Herstellung von 1-Methyl-2-[N-[4-(1,2,4-Oxadiazol-5-on-3-yl)phenyl]-amino-methyl]-benzimidazo1-5-yl-carbonsäure-N-(2-pyridyl)-N-(2-ethoxycarbonylethyl)-amid (3):

11,35 g (70 mmol) 1,1 '-Carbonyldiimidazol werden in 100 ml THF suspendiert und auf 50°C erwärmt. 14,23 g (60,5 mmol) **(2B)** werden portionsweise zugegeben. 17,1 g (50 mmol) AMPBA werden in 37 ml THF unter Erwärmen auf 50°C gelöst.
Nach ca. 90 min wird die Suspension zur Lösung von AMPBA zudosiert und mit 20 ml THF nachgespült. Die Reaktionsmischung wird ca. 18 h gerührt und anschließend nach

Zugabe von 100 ml Essigsäure auf Rückfluss erhitzt, sodass das THF abdestilliert. Nach ca. 1 h wird mit 400 ml Wasser versetzt und gerührt.

Die Lösung wird abgekühlt, die ausgefallene rosafarbene Festsubstanz abfiltriert und mit 20 ml Wasser nach gewaschen in 2 Portionen und bei max 50°C im Vakuum getrocknet. Die isolierte Substanz stellt das Diacetat von (3) dar.

Ausbeute: 24,8 g Substanz (75% d.Th.); Schmelzpunkt: ab 167°C unter Zers. (DSC); Reinheit: > 95% HPLC-Peakfläche

### Beispiel 4 - Herstellung von 1-Methyl-2-[N-[4-amidinophenyl]-amino-methyl]-benzimidazol-5-yl-carbonsäure-N-(2-pyridyl)-N-(2-ethoxycarbonylethyl)-amid (4)

37,3 g (56,4 mmol) (3) werden in 900 ml Ethanol gelöst und nach Zugabe von 10 ml Essigsäure und bei 2 bar Wasserstoff mit 4 g wasserfeuchtem 10% Pd/C bei RT hydriert. Man filtriert vom Katalysator und gibt 17 g (89,4 mmol) PTSA, gelöst in 180 ml Ethanol zu. Das Tosylat von **(4)** fällt aus, wird abfiltriert und in mehreren Portionen mit 150 ml Ethanol nachgewaschen. Man erhält feuchte Substanz, die bei 35°C im Vakuum getrocknet wird.

Ausbeute: 34,5 g hellbeige Substanz (91,3% d.Th.); Schmelzpunkt: 187°C (DSC); Reinheit: > 98% HPLC-Peakfläche.

### Beispiel 5 - Herstellung von 1-Methyl-2-[N-[4-(N-n-hexyloxycarbonylamidino) phenyl]-amino-methyl]-benzimidazol-5-yl-carbonsäure-N-(2-pyridyl)-N-(2-ethoxycarbonylethyl)-amid (5)

Die nach Beispiel 4 erhaltene Verbindung wird in an sich bekannter Weise mit Hexylchloroformiat in Gegenwart einer Base umgesetzt.

## Patentansprüche

1. Verfahren zur Herstellung eines gegebenenfalls substituierten 4-Benzimidazol-2-ylmethylamino)-benzamidins der Formel (I) 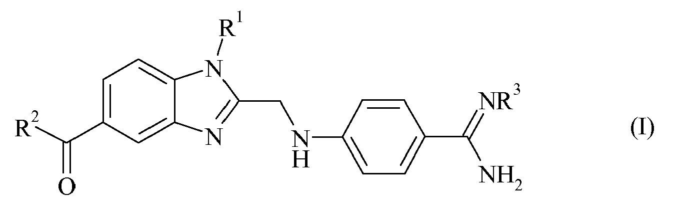 worin
R¹ für eine Methylgruppe steht,
R² für eine R²¹NR²²-Gruppe steht, in der
R²¹ eine Ethylgruppe, die durch eine Ethoxycarbonylgruppe substituiert ist, bedeutet, und
R²² Pyridin-2-ylgruppe bedeutet, und
R³ für eine Hexyloxycarbonylgruppe steht,
wobei man im Schritt (a) ein Phenyldiamin der Formel (II) 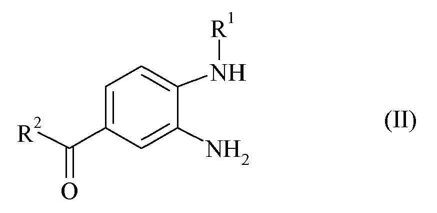
worin R¹ und R² die für Formel (I) angegebene Bedeutung aufweisen, mit 2-[4-(1,2,4-Oxadiazol-5-on-3-yl)-phenylamino]-essigsäure umsetzt; das erhaltene Produkt der Formel (III) 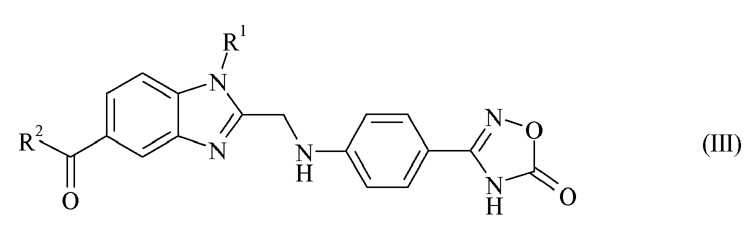
worin R¹ und R² die für Formel (I) angegebene Bedeutung aufweisen,
in Schritt (b) hydriert, und
(c) gegebenenfalls die so erhaltene Verbindung der Formel (I), worin R³ Wasserstoff bedeutet, mit einer Verbindung der Formel (IV)
R³-X (IV)
worin R³ die für Formel (I) angegebene Bedeutung aufweist, und X für eine geeignete Abgangsgruppe steht,
umsetzt.

2. Verfahren nach einem der vorangehenden Ansprüche, **dadurch gekennzeichnet, dass** die Kondensation des Schrittes (a) in Gegenwart eines inerten Verdünnungsmittels und eines wasserbindenden Mittels durchführt.

3. Verfahren nach einem der vorangehenden Ansprüche, **dadurch gekennzeichnet, dass** man die Hydrierung des Schrittes (b) in Gegenwart eines inerten Verdünnungsmittels und eines Hydrierkatalysators durchführt.

4. Verfahren nach einem der vorangehenden Ansprüche, **dadurch gekennzeichnet, dass** man zur Herstellung von 2-[4-(1,2,4-Oxadiazol-5-on-3-yl)-phenylamino]-essigsäure 2-[4-(1,2,4-Oxadiazol-5-on-3-yl)-anilin mit einem 2-Halogenessigsäureester in Gegenwart einer schwachen Base umsetzt, und den erhaltenen 2-[4-(1,2,4-Oxadiazol-5-on-3-yl)-phenylamino]-essigsäureester verseift.

5. Verfahren nach einem der vorangehenden Ansprüche, **dadurch gekennzeichnet, dass** man zur Herstellung von 2-[4-(1,2,4-Oxadiazol-5-on-3-yl)-anilin 4-Aminophenyl-amidoxim mit einem Dialkylcarbonat in Gegenwart einer Base umsetzt.

6. Verfahren nach einem der vorangehenden Ansprüche, **dadurch gekennzeichnet, dass** man die so erhaltene Verbindung der Formel (I) anschließend in ein physiologisch verträgliches Salz um wandelt.

7. Verfahren nach Anspruch 6, **dadurch gekennzeichnet, dass** das physiologisch verträgliche Salz das Methansulfonat ist.

8. Verbindung der Formel (III) 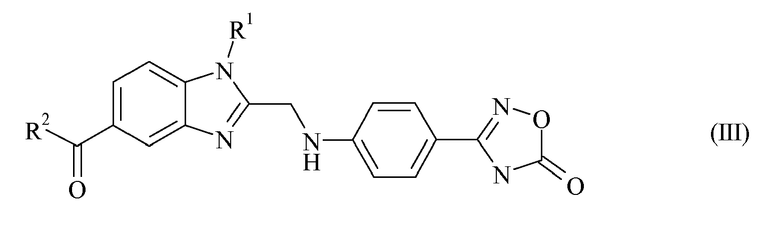 worin R¹ und R² die in Anspruch 1 angegebene Bedeutung aufweisen.

9. 2-[4-(1,2,4-Oxadiazol-5-on-3-yl)-phenylamino]-essigsäure.

## Claims

1. Process for preparing an optionally substituted 4-benzimidazol-2-ylmethylamino)-benzamidine of formula (I) 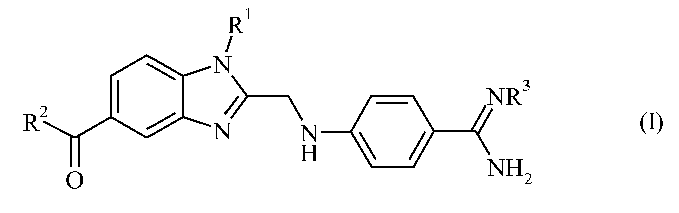 wherein
R¹ denotes a methyl group,
R² denotes an R²¹NR²² group, wherein
R²¹ denotes an ethyl group which is substituted by an ethoxycarbonyl group, and
R²² denotes a pyridin-2-yl group, and
R³ denotes a hexyloxycarbonyl group,
wherein in step (a) a phenyldiamine of formula (II) 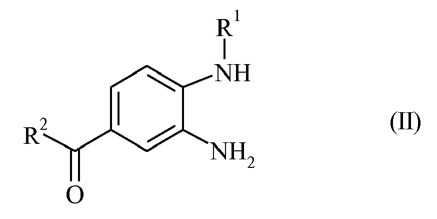
wherein R¹ and R² have the meanings given for formula (I),
is reacted with 2-[4-(1,2,4-oxadiazol-5-on-3-yl)-phenylamino]-acetic acid; the resulting product of formula (III) 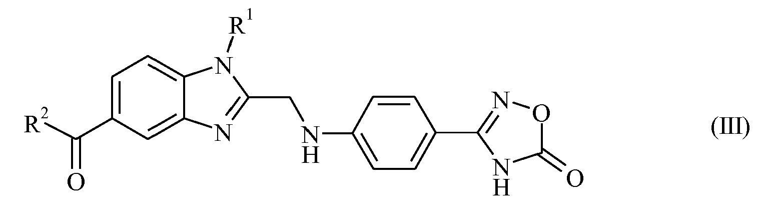
wherein R¹ and R² have the meanings given for formula (I),
is hydrogenated in step (b), and
(c) the compound of formula (I) thus obtained, wherein R³ denotes hydrogen, is optionally reacted with a compound of formula (IV)
R³-X (IV)
wherein R³ has the meaning given for formula (I), and X denotes a suitable leaving group.

2. Method according to one of the preceding claims, **characterised in that** the condensation of step (a) is carried out in the presence of an inert diluent and a water-binding agent.

3. Method according to one of the preceding claims, **characterised in that** the hydrogenation of step (b) is carried out in the presence of an inert diluent and a hydrogenation catalyst.

4. Method according to one of the preceding claims, **characterised in that**, in order to prepare 2-[4-(1,2,4-oxadiazol-5-on-3-yl)-phenylamino]-acetic acid, 2-[4-(1,2,4-oxadiazol-5-on-3-yl)-aniline is reacted with a 2-haloacetic acid ester in the presence of a weak base, and the 2-[4-(1,2,4-oxadiazol-5-on-3-yl)-phenylamino]-acetic acid ester obtained is saponified.

5. Method according to one of the preceding claims, **characterised in that**, in order to prepare 2-[4-(1,2,4-oxadiazol-5-on-3-yl)-aniline, 4-aminophenylamidoxime is reacted with a dialkyl carbonate in the presence of a base.

6. Method according to one of the preceding claims, **characterised in that** the compound of formula (I) thus obtained is then converted into a physiologically acceptable salt.

7. Method according to claim 6, **characterised in that** the physiologically acceptable salt is the methanesulphonate.

8. Compound of formula (III) 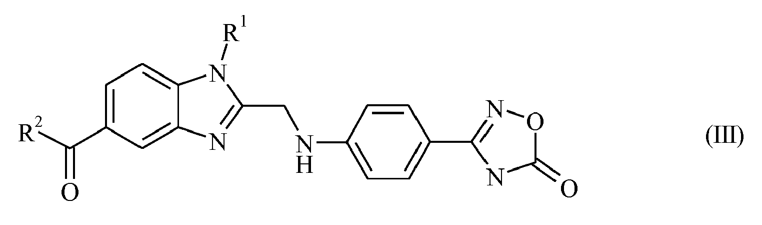 wherein R¹ and R² have the meanings given in claim 1.

9. 2-[4-(1,2,4-Oxadiazol-5-on-3-yl)-phenylamino]-acetic acid.

## Revendications

1. Procédé de production d'une 4-benzimidazol-2-ylméthylamino)-benzamidine, éventuellement substituée, de formule (I) 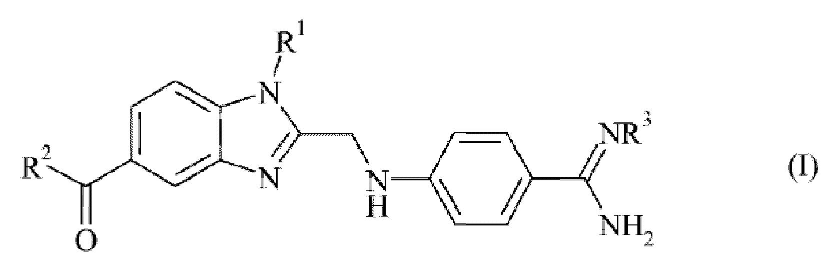 dans laquelle
R¹ représente un groupe méthyle,
R² représente un groupe R²¹NR²² dans lequel
R²¹ désigne un groupe éthyle qui est substitué par un groupe éthoxycarbonyle, et
R²² désigne un groupe pyridin-2-yle, et
R³ représente un groupe hexyloxycarbonyle,
dans lequel on fait réagir à l'étape (a) une phényldiamine de formule (II) 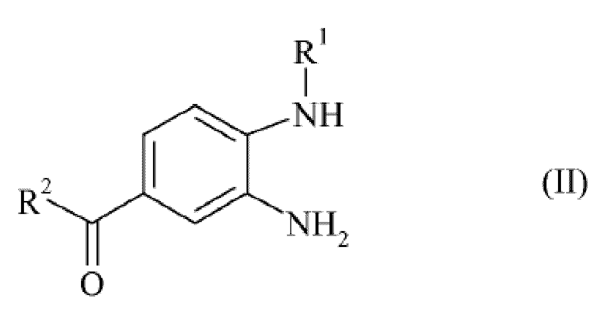
dans laquelle R¹ et R² présentent la signification indiquée pour la formule (I),
avec de l'acide 2-[4-(1,2,4-oxadiazol-5-on-3-yl)-phénylamino]-acétique ;
à l'étape (b), on hydrogène le produit de formule (III) obtenu 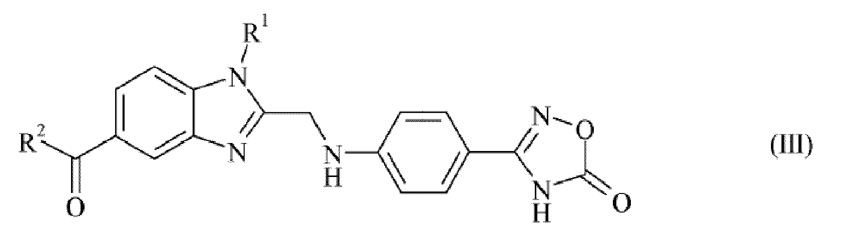
dans laquelle R¹ et R² présentent la signification indiquée pour la formule (I), et
(c) éventuellement, on fait réagir le composé de formule (I) ainsi obtenu, dans laquelle R³ désigne un atome d'hydrogène, avec un composé de formule (IV)
R³-X (IV)
dans laquelle R³ présente la signification indiquée pour la formule (I) et X représente un groupe partant approprié.

2. Procédé selon l'une des revendications précédentes, **caractérisé en ce que** la condensation de l'étape (a) s'effectue en présence d'un diluant inerte et d'un agent fixant l'eau.

3. Procédé selon l'une des revendications précédentes, **caractérisé en ce que** l'on réalise l'hydrogénation de l'étape (b) en présence d'un diluant inerte et d'un catalyseur d'hydrogénation.

4. Procédé selon l'une des revendications précédentes, **caractérisé en ce que**, pour la production de l'acide 2-[4-(1,2,4-oxadiazol-5-on-3-yl)-phénylamino]-acétique, on fait réagir de la 2-[4-(1,2,4-oxadiazol-5-on-3-yl)-aniline avec un ester d'acide 2-halogéno-acétique en présence d'une base faible et on saponifie l'acide 2-[4-(1,2,4-oxadiazol-5-on-3-yl)-phénylamino]-acétique obtenu.

5. Procédé selon l'une des revendications précédentes, **caractérisé en ce que**, pour la production de la 2-[4-(1,2,4-oxadiazol-5-on-3-yl)-aniline, on fait réagir du 4-aminophényl-amidoxime avec un carbonate de dialkyle en présence d'une base.

6. Procédé selon l'une des revendications précédentes, **caractérisé en ce qu'**on convertit ensuite le composé de formule (I) ainsi obtenu en un sel physiologiquement acceptable.

7. Procédé selon la revendication 6, **caractérisé en ce que** le sel physiologiquement acceptable est le méthanesulfonate.

8. Composé de formule (III) 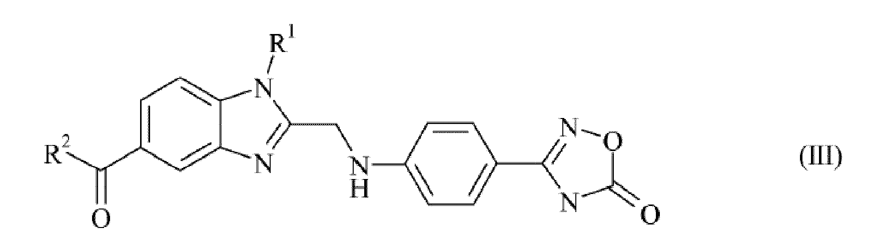 dans laquelle R¹ et R² présentent la signification indiquée dans la revendication 1.

9. Acide 2-[4-(1,2,4-oxadiazol-5-on-3-yl)-phénylamino]-acétique.
